Europäisches Patentamt

European Patent Office

Office européen des brevets

(11) Veröffentlichungsnummer: **0 066 701**
**B1**

(12) **EUROPÄISCHE PATENTSCHRIFT**

(45) Veröffentlichungstag der Patentschrift: 28.09.88

(21) Anmeldenummer: 82103443.6

(22) Anmeldetag: 23.04.82

(51) Int. Cl.⁴: **C 12 N 15/00** // C12R1/465

(54) **Plasmid p SG 2 und Verfahren zu seiner Gewinnung.**

(30) Priorität: 30.04.81 DE 3117131

(43) Veröffentlichungstag der Anmeldung:
15.12.82 Patentblatt 82/50

(45) Bekanntmachung des Hinweises auf die
Patenterteilung:
28.09.88 Patentblatt 88/39

(84) Benannte Vertragsstaaten:
AT BE CH DE FR GB IT LI LU NL SE

(56) Entgegenhaltungen:
THE JOURNAL OF ANTIOBIOTICS, Band 33, Nr.
1, Januar 1980, Seiten 88-91, Tokyo, JP.
M.OKANISHI et al.: "Isolation and
characterizationof plasmid DNAs in
actinomycetes"

THE JOURNAL OF ANTIBIOTICS, Band 32, Nr.
12, Dezember 1979, Seiten 1348-1350, Takeda
Chemical Industries, Ltd., Osaka, JP.
T.HAYAKAWA et al.: "Isolation and
characterizationof plasmids from
streptomyces"

(73) Patentinhaber: HOECHST
AKTIENGESELLSCHAFT
Postfach 80 03 20
D-6230 Frankfurt am Main 80 (DE)

(72) Erfinder: Pühler, Alfred, Prof. Dr.
Am Waldschlösschen 2
D-4800 Bielefeld (DE)
Erfinder: Wohlleben, Wolfgang, Dr.
Heidelberger Weg 7
D-4800 Bielefeld (DE)
Erfinder: Leineweber, Michael, Dr.
Sachsenring 10
D-6238 Hofheim am Taunus (DE)

**Beschreibung**

Gegenstand der Erfindung ist das Plasmid p SG 2 und ein Verfahren zu seiner Gewinnung aus einer Kultur von Streptomyces ghanaensis ATCC 14 672.

Es ist aus der deutschen Offenlegungsschift 30 05 226 bekannt, daß aus einer Kultur von Streptomyces espinosus das Plasmid p UC 6 gewonnen werden kann. Es ist weiterhin aus der PCT-Anmeldung 79/01169 bekannt, daß aus Streptomyces lividans ein Plasmid gewonnen werden kann, welches als Vektor für die Einführung von DNA in Mikroorganismen geeignet erscheint.

Hieraus ist erkennbar, daß der Anwendung gentechnologischer Methoden auf Antibiotika bildende Streptomyces-Arten besondere Bedeutung beigemessen wird. Jedoch sind die bisher bekannten Verfahren für die praktische Anwendung von geringem Interesse geblieben.

Die genetische Verbesserung der für die Antibiotika-Herstellung verwendeten Streptomyceten-Stämme mit dem Ziel einer Steigerung der Menge des gebildeten Antibiotikums ist eine wichtige Aufgabe. Sie läßt sich jedoch nur dann erfolgreich mit gentechnologischen Methoden lösen, wenn ein Plasmid zur Verfügung steht, das von der als Wirtszelle eingesetzten Streptomyces-Art nicht alsbald wieder eliminiert wird, wie es bei miteinander nicht verwandten Arten häufig beobachtet wird.

Es stellte sich deshalb die Aufgabe, ausreichende Mengen eines Plasmids zu gewinnen, das als Vektor zur genetischen Verbesserung von Streptomyces-ghanaensis-Stämmen geeignet ist.

Es hat sich nun gezeigt, daß diese Aufgabe gelöst wird durch das Plasmid p SG 2, welches aus einer Kultur von Streptomyces ghanaensis ATCC 14 672 gewonnen wird, ein Molekulargewicht von 9,2 Megadalton, eine Konturlänge von 4,58 μm und eine Moleküllänge von etwa 13,8 Kilobasen (= kb) aufweist.

Der hier verwendete Stamm von Streptomyces ghanaensis ist im einzelnen in der deutschen Patentschrift 1 113 791 beschrieben und wird dort bezeichnet als S. ghanaensis nov. sp. 4092. In einer Kultur von Streptomyces ghanaensis liegen pro Zelle etwa 10 bis 20 Kopien des Plasmids p SG 2 vor. Dieses Plasmid ist somit ein geeignetes Ausgangsplasmid für die Anwendung gentechnologischer Methoden sowohl auf den Stamm Streptomyces ghanaensis selbst als auch auf andere Streptomyces-Arten.

Das Plasmid p SG 2 besitzt ein Molekulargewicht von etwa 9,2 Megadalton und eine Moleküllänge von ungefähr 13,8 Kilobasen. Der Nachweis dafür wurde mit der Agarosegel-Elektrophorese des Moleküls selbst und seiner Spaltstücke geführt. Die Längenbestimmung von 4,58 ± 0,09 μm ergibt sich aus der elektronenmikroskopischen Vermessung von 27 Plasmidmolekülen. Die Intensität der Plasmidbande in der Elektrophorese erlaubt die Abschätzung der Kopienzahl pro Zelle auf etwa 10 bis 20 Stück. Beim Fragmentieren des Plasmids p SG 2 mit einer größeren Zahl bekannter Restriktions-Endonukleasen wurden die Spaltstellen sowie die Fragmentlängen bestimmt und sind in der folgenden Tabelle zusammengefaßt:

Restriktionsverhalten von p SG 2 bei Verdauung mit verschiedenen Enzymen:

| Restriktions-enzym | Zahl der Schnitt stellen | Länge der Fragmente | | Gesamtlänge | |
|---|---|---|---|---|---|
| EcoRI | – | | | | |
| BamHI | – | | | | |
| SalI | – | | | | |
| HpaI | – | | | | |
| HindII | – | | | | |
| HindIII | 1 | ∼14 | kb | ∼14 | kb |
| BglII | 2 | 11,25 | kb | 13,85 | kb |
| | | 2,6 | kb | | |
| ClaI | 2 | 10,15 | kb | 13,8 | kb |
| | | 3,65 | kb | | |
| PstI | 2 | 10,85 | kb | 13,85 | kb |
| | | 3,0 | kb | | |
| BclI | 3 | 11,6 | kb | 13,85 | kb |
| | | 1,25 | kb | | |
| | | 1,0 | kb | | |
| BstEII | 8 | 3,8 | kb | 13,65 | kb |
| | | 2,45 | kb | | |
| | | 1,9 | kb | | |
| | | 1,15 | kb | | |
| | | 1,15 | kb | | |
| | | 1,0 | kb | | |
| | | 0,9 | kb | | |
| | | 0,8 | kb | | |
| SmaI | 10 | | | | |
| XhoI | 10 | nicht be- | | | |
| Sau3A | 10 | stimmt | | | |

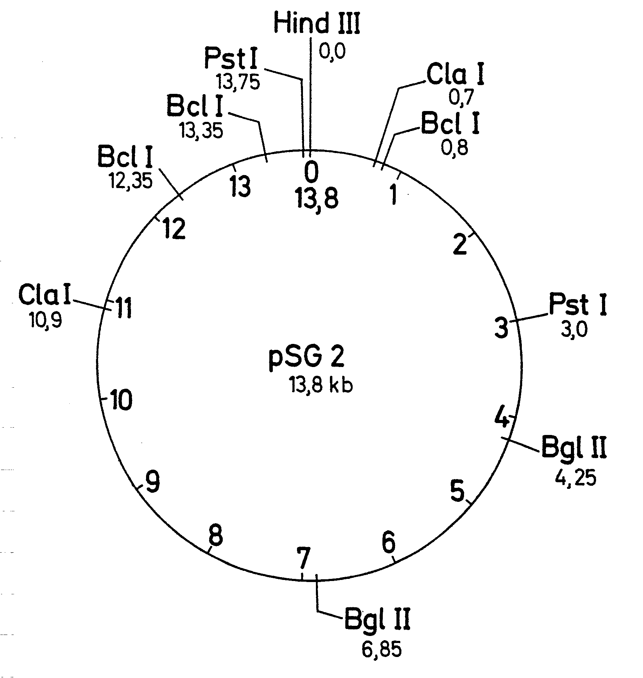

Ein Restriktions-Endonucleasen-Spaltbild von p SG 2 zeigt die beigefügte Zeichnung. Sie läßt jeweils ein langes und ein kurzes PstI-bzw. ClaI und Bgl II-Fragment sowie ein langes und zwei kurze Bcl I-Fragmente erkennen. Die Lage der Schnittstellen zueinander wurde durch Teil- und Doppelverdauungen bestimmt.

Das Plasmid p SG 2 ist also zusätzlich .dadurch gekennzeichnet, daß es von den Restriktions-Endonukleasen EcoR I, BamH I, Sal I, Hpa I und Hind II überhaupt nicht angegriffen wird, jedoch von der Restriktions-Endonuklease Hind III in ein Fragment einer Länge von etwa 14 kb, von Cla I in zwei Fragmente mit den Längen 10,15 und 3,65 kb und von Pst I in zwei Fragmente mit den Längen 10,85 und 3,0 kb, von Bgl II in zwei Fragmente mit den Längen 11,25 kb und 2,6 kb, sowie von Bcl I in drei Fragmente mit den Längen 11,6 kb, 1,25 kb und 1,0 kb zerlegt wird.

Dieses Schnittmuster verdeutlicht, daß das Plasmid p SG 2 zur Konstruktion eines Vektorplasmids verwendet werden kann. Bei den Arbeiten zur genetischen Veränderung dieses Plasmids können die gleichen Verfahren verwendet werden, die bei Escherichia coli-Plasmiden erfolgreich eingesetzt wurden und z.B. beschrieben sind von M. Bibb, J. C. Schottel, S. N. Cohen, Nature 284, 526—531 (1980) und C. J. Thompson, J. M. Ward, D. A. Hopwood, Nature 286, 525—527 (1980).

So können in die Hind III-, Cla I-, Bgl II, Pst I- und Bcl I-Schnittstellen ohne weiteres Antibiotika-Resistenzen eingefügt werden. Weiterhin erscheint es möglich, in das Plasmid p SG 2 Gene einzuführen, die in Streptomyces ghanaensis zu einer Steigerung der Antibiotika-Produktion führen. Hierbei kommen sowohl Gene für den Biosyntheseweg als auch Regulationsgene in Frage. Derartig modifizierte p SG 2-Plasmide sind nach allen bisher vorliegenden Beobachtungen in Streptomyces-Zellen ebenso lebens- und vermehrungsfähig wie das Ausgangsplasmid.

Das Plasmid p SG 2 erhält man aus einer Kultur von Streptomyces ghanaensis ATCC 14 672 durch Züchten der Kultur auf einem geeigneten Medium, Fragmentieren des Myzels, Inkubieren des fragmentierten Myzels, Ernten der Kultur und anschließendes Lysieren des Myzels. Die Isolierung des Plasmids aus dem Lysat gelingt durch eine alkalische Denaturierung, bei der man eine etwa 0,1—0,5 normale Natronlauge, der zweckmäßigerweise etwa 1% Natriumdodecylsulfat zugesetzt wird, bei etwa 0°C bis zu 10 Minuten lang auf das Lysat einwirken läßt. Danach wird durch Zugabe einer Pufferlösung wieder ein schwach saurer pH eingestellt und damit eine Renaturierung erreicht. Anschließend wird die Mischung zentrifugiert und aus dem Überstand durch Zugabe eines Überschusses von Äthanol bei −20°C und anschließendes Zentrifugieren ein Pellet gewonnen. Dieses Pellet wird dann in einer Pufferlösung aufgenommen und in einer Cäsiumchlorid-Ethidiumbromid-Lösung einer isopyknischen Dichtegradientenzentrifugation in der Ultrazentrifuge unterworfen. Dann werden die Plasmide aus dem Gradienten durch Punktieren entnommen, chromatographisch gereinigt und im Agarosegel und im Elektronenmikroskop charakterisiert. Es handlet sich hierbei um bekannte Methoden (vgl. R. Radloff, W. Bauer, J. Vinograd, Proc. Natl. Acad. Sci. USA, 57, 1514—1521 (1967) und A. A. Szalay, C. J. Mackey, H. W. R. Langridge, Enzyme Microb. Technol. *1*, 154—164 (1976).

Hier und im folgenden beziehen sich Prozentangaben auf das Gewicht, sofern nichts anderes angegeben ist.

Beispiel 1

Zur Herstellung des Plasmids p SG 2 wurde folgendermaßen verfahren:

Eine Kultur von Streptomyces ghanaensis ATCC 14 672 wurde 3 bis 5 Tage lang bei 30°C in 100 ml eines Nährmediums folgender Zusammensetzung angezüchtet:

| | |
|---|---|
| Glukose | 1 g |
| Pepton | 0,4 g |
| Hefe-Extrakt | 0,4 g |
| $MgSO_4 \cdot 7H_2O$ | 0,05 g |
| $KH_2PO_4$ | 0,2 g |
| $K_2H PO_4$ | 0,4 g |
| Glycin | 2 g |
| Aq. dest. | ad 100 g |

Der Kulturüberstand wurde dann abgekippt, die Restkultur homogenisiert und dann 5 Minuten lang zentrifugiert. Nach dem zweimaligen Waschen in einer Pufferlösung, bestehend aus 10 mmol Tris-HCl und 1 mmol Äthylendiamintetraessigsäure (pH 7,5), der noch 10% Sucrose zugesetzt waren, wurde in 10 ml einer Lösung folgender Zusammensetzung resuspendiert:

50 mmol Glukose
10 mmol EDTA
25 mmol Tris-HCl (Tris-(hydroxymethyl)-aminomethan)
  2 mg/ml Lysozym

Diese Lösung hat pH 8.

Nach einer Inkubationszeit bei 32°C von 30 bis 60 Min. wurden 20 ml einer frisch zubereiteten 0,2 normalen NaOH, der noch 1% Natriumdodecylsulfat beigefügt war, zugegeben und die Mischung bei 0°C 5 Minuten stehen gelassen. Nach Zugabe von 15 ml einer 3-molaren Natriumazetatlösung von einem pH 4,8 wurde erneut gemischt und 1 Stunde bei 0°C inkubiert. Nach erneuter Zentrifugation wurde der Überstand mit dem 3fachen Volumen Äthanol (96%) bei −20°C versetzt. Der Niederschlag wurde durch Zentrifugieren abgetrennt, im Vakuum getrocknet und dann in 6 ml eines Puffers von 10 mmol Tris-HCl und 1 mmol EDTA (pH 7,5) aufgenommen. Dann wurde in der Ultrazentrifuge 2 Tage bei 34000 rpm im Cäsiumchlorid/Ethidiumbromid-Gradienten zentrifugiert. Die die Plasmide enthaltende Fraktion wurde anschließend noch an Apatit chromatographiert.

Auf diese Weise wurde das Plasmid p SG 2 in reiner Form isoliert.

## Beispiel 2

Das Plasmid p SG 2 konnte auch auf folgende Weise gewonnen werden:

1 g Myzel von Streptomyces ghanaensis ATCC 14 672 wurde 3 bis 5 Tage bei 30°C in dem gleichen Medium angezüchtet, das in Beispiel 1 beschrieben wurde. Auch hier wurde der Kulturüberstand abgegossen, homogenisiert und das Zellmaterial durch Zentrifugation abgetrennt. Dann wurde zweimal gewaschen mit einem Puffer, der aus 10 mmol Tris-HCl und 140 mmol NaCl (pH 8,0) bestand. Anschließend wurde resuspendiert in 5 ml eines Puffers bestehend aus 100 mmol Tris-HCl, 20 mmol EDTA und 25% Sucrose. Dann wurden 0,1 ml einer Lysozymlösung (30 mg/ml) zugegeben und 20 Min. bei 25°C inkubiert. Nach Abkühlung auf 0°C und Zugabe 6 ml eiskalten Wassers wurde 1 ml einer 1%igen Lösung von Natriumdodecylsulfat zugegeben und nach Zufügung von weiteren 3,4 ml einer 5 molaren NaCl Lösung vorsichtig gemischt. Der Ansatz wurde dann bei 4°C stehen gelassen.

Nachdem die Ausfällung bei 4°C beendet war, wurde 1 Stunde bei 0°C und 16000 rpm zentrifugiert. Der Überstand wurde mit einem Drittel seines Volumens einer 42%igen Polyäthylenglykollösung versetzt, (Molekulargewicht des Polyäthylenglykols 6000), wobei ein Niederschlag entstand. Nachdem die Ausfällung bei einer Temperatur von 4°C beendet war, wurden die Plasmide durch Zentrifugieren bei 16000 rpm während 15 Minuten abgetrennt und dann das gebildete Pellet vorsichtig in 1 ml einer 0,4%igen Lösung von Sarkosyl* in einem Puffer, bestehend aus 10 mmol Tris-HCl und 1 mmol EDTA (pH 7,5) aufgenommen. Mit dem gleichen Puffer wurde schließlich auf 5 ml aufgefüllt und in der Ultrazentrifuge im Cäsiumchlorid-Ethidiumbromid-Gradienten zentrifugiert. Danach wurde wie in Beispiel 1 weiter gearbeitet.

### Patentansprüche für die Vertragsstaaten: BE CH DE FR GB IT LI LU NL SE

1. Plasmid p SG 2, dadurch gekennzeichnet, daß es aus einer Kultur von Streptomyces ghanaensis ATCC 14 672 erhältlich ist und eine Konturlänge von 4,58 µm und eine Moleküllänge von etwa 13,8 Kilobasen (= kb) aufweist und daß es von den Restriktions-Endonukleasen EcoR I, BamH I, Sal I, Hpa I und Hind II nicht fragmentiert wird, jedoch von der Restriktions-Endonuklease Hind III in ein Fragment einer Länge von etwa 14 kb, von Cla I in zwei Fragmente mit den Längen 10,15 und 3,65 kb und von Pst I in zwei Fragmente mit den Längen 10,85 und 3,0 kb, von Bgl II in zwei Fragmente mit den Längen 11,25 kb und 2,6 kb, sowie von Bcl I in drei Fragmente mit den Längen 11,6 kb, 1, 25 kb und 1,0 kb zerlegt wird.

2. Verfahren zur Gewinnung des Plasmids p SG 2, dadurch gekennzeichnet, daß man eine Kultur von Streptomyces ghanaensis ATCC 14672 lysiert und das Plasmid isoliert.

3. Verfahren nach Anspruch 2, dadurch gekennzeichnet, daß man das Plasmid durch eine alkalische Denaturierung mit anschließender Renaturierung und Alkoholfällung aus dem Lysat isoliert.

4. Verfahren nach Anspruch 2 oder 3, dadurch gekennzeichnet, daß man die Kultur von Streptomyces ghanaensis ATCC 14672 bei einer Temperatur von etwa 0°C durch ein Detergens in einer Menge von etwa 0,1 Gew.-% lysiert.

5. Verwendung des Plasmids p SG 2 zur Konstruktion von Vektorplasmiden.

### Patentansprüche für den Vertragsstaat: AT

1. Verfahren zur Gewinnung des Plasmids p SG 2, das eine Konturlänge von 4,58 µm und eine Moleküllänge von etwa 13,8 Kilobasen (= kb) aufweist und von den Restriktions-Endonukleasen EcoR I, BamH I, Sal I, Hpa I und Hind II nicht fragmentiert wird, jedoch von der Restriktions-Endonuklease Hind III in ein Fragment einer Länge von etwa 14 kb, von Cla I in zwei Fragmente mit den Längen 10,15 und 3,65 kb und von Pst I in zwei Fragmente mit den Längen 10,85 und 3,0 kb, von Bgl II in zwei Fragmente mit den Längen 11,25 kb und 2,6 kb, sowie von Bcl I in drei Fragmente mit den Längen 11,6 kb, 1, 25 kb und 1,0 kb zerlegt wird, dadurch gekennzeichnet, daß man eine Kultur von Streptomyces ghanaensis ATCC 14672 lysiert und das Plasmid isoliert.

2. Verfahren nach Anspruch 1, dadurch gekennzeichnet, daß man das Plasmid durch eine alkalische Denaturierung mit anschließender Renaturierung und Alkoholfällung aus dem Lysat isoliert.

3. Verfahren nach Anspruch 1 oder 2, dadurch gekennzeichnet, daß man die Kultur von Streptomyces ghanaensis ATCC 14672 bei einer Temperatur von etwa 0°C durch ein Detergens in einer Menge von etwa 0,1 Gew.-% lysiert.

4. Verwendung des Plasmids p SG 2 zur Konstruktion von Vektorplasmiden.

### Revendications pour les Etats contractants: BE CH DE FR GB IT LI LU NL SE

1. Plasmide p SG 2 caractérisé en ce qu'il peut être obtenu à partir d'une culture de Streptomyces ghanaensis ATCC 14 672 et qu'il présente une longueur de contour de 4,58 µm et une longueur de molécule d'environ 13,8 kilobases (= kb), qu'il n'est pas fragmenté par les endonucléases de restriction EcoR I, BamH I, Sal I, Hpa I et Hind II et qu'il est cependant décomposé par l'endonucléase de restriction Hind III en un fragment d'une longueur d'environ 14 kb, par Cla I en deux fragments de longueurs 10,15 et 3,65 kb, par Pst

*(N-Lauroyl-sarkosid)

I en deux fragments de longueurs 10,85 et 3,0 kb, par Bgl II en deux fragments de longueurs 11,25 kb et 2,6 kb ainsi que par Bcl I en trois fragments de longueurs 11,6 kb, 1,25 kb et 1,0 kb.

2. Procédé d'obtention du plasmide p SG 2 caractérisé en ce qu'un lyse une culture de Streptomyces ghanaensis ATCC 14 672 et on isole le plasmide.

3. Procédé selon la revendication 2 caractérisé en ce que l'on isole le plasmide à partir du lysat par une dénaturation alcaline suivie d'une renaturation et d'une précipitation à l'alcool.

4. Procédé selon la revendication 2 ou 3 caractérisé en ce qu'on lyse la culture de Streptomyces ghanaensis ATCC 14 672 à une température d'environ 0°C par un détérgent en quantité d'environ 0,1% en poids.

5. Utilisation du plasmide p SG 2 pour la construction de plasmides vecteurs.

**Revendications pour l'Etat contractant: AT**

1. Procédé d'obtention du plasmide p SG 2 qui présente une longueur de contour de 4,58 μm et une longueur de molécule d'environ 13,8 kilobases (= kb), et n'est pas fragmenté par les endonucléases de restriction EcoR I, BamH I, Sal I, Hpa I et Hind II et est cependant décomposé par l'endonucléase de restriction Hind III en un fragment d'une longueur d'environ 14 kb, par Cla I en deux fragments de longueurs 10,15 et 3,65 kb, par Pst I en deux fragments de longueurs 10,85 et 3,0 kb, par Bgl II en deux fragments de longueurs 11,25 kb et 2,6 kb ainsi que par Bcl I en trois fragments de longueurs 11,6 kb, 1,25 kb et 1,0 kb, caractérisé en qu'on lyse une culture de Streptomyces ghanaensis ATCC 14 672 et on isole le plasmide.

2. Procédé selon la revendication 1 caractérisé en ce qu'on isole le plasmide à partir du lysat par une dénaturation alcaline suivie d'une renaturation et d'une précipitation à l'alcool.

3. Procédé selon la revendication 1 ou 2 caractérisé en ce qu'on lyse la culture de Streptomyces ghanaensis ATCC 14 672 à une température d'environ 0°C par un détérgent en quantité d'environ 0,1% en poids.

4. Utilisation du plasmide p SG 2 pour la construction de plasmides vecteurs.

**Claims for the Contracting States: BE CH DE FR GB IT LI LU NL SE**

1. The plasmid p SG 2, characterized in that it is obtained from a culture of Streptomyces ghanaensis ATCC 14672 and has a contour length of 4.58 μm and a molecular length of about 13.8 kilobases (= kb) and is not fragmented by the restriction endonucleases EcoR I, BamH I, Sal I, Hpa I and Hind II, but is cleaved by the restriction endonuclease Hind III into a fragment of length of about 14 kb, by Cla I into two fragments of lengths 10.15 and 3.65 kb and by Pst I into two fragments of lengths 10.85 and 3.0 kb, by Bgl II into two fragments of lengths 11.25 kb and 2.6 kb, and by Bcl I into three fragments of lengths 11.6 kb, 1.25 kb and 1.0 kb.

2. A process for obtaining the plasmid p SG 2, characterized in that a culture of Streptomyces ghanaensis ATCC 14672 is lysed, and the plasmid is isolated.

3. The process according to claim 2, characterized in that the plasmid is isolated from the lysate by alkaline denaturing with subsequent renaturing and alcohol precipitation.

4. The process according to claim 2 or 3, characterized in that the culture of Streptomyces ghanaensis ATCC 14672 is lysed at a temperature of about 0°C by a detergent in an amount of about 0.1% by weight.

5. The use of the plasmid p SG 2 for construction of vector plasmids.

**Claims for the Contracting State: AT**

1. A process for obtaining the plasmid p SG 2, which has a contour length of 4.58 μm and a molecular length of about 13.8 kilobases (= kb) and is not fragmented by the restriction endonucleases EcoR I, BamH I, Sal I, Hpa I and Hind II, but is cleaved by the restriction endonuclease Hind III into a fragment of length of about 14 kb, by Cla I into two fragments of lengths 10.15 and 3.65 kb and by Pst I into two fragments of lengths 10.85 and 3.0 kb, by Bgl II into two fragments of lengths 11.25 kb and 2.6 kb, and by Bcl I into three fragments of lengths 11.6 kb, 1.25 kb and 1.0 kb, characterized in that a culture of Streptomyces ghanaensis ATCC 14672 is lysed, and the plasmid is isolated.

2. The process according to claim 1, characterized in that the plasmid is isolated from the lysate by alkaline denaturing with subsequent renaturing and alcohol precipitation.

3. The process according to claim 1 or 2, characterized in that the culture of Streptomyces ghanaensis ATCC 14672 is lysed at a temperature of about 0°C by a detergent in an amount of about 0.1% by weight.

4. The use of the plasmid p SG 2 for construction of vector plasmids.

pSG 2
13,8 kb